# EUROPEAN PATENT SPECIFICATION

(11) **EP 4 489 827 B1**
(45) Date of publication and mention of the grant of the patent: **28.10.2026**
(21) Application number: 23708571.7
(22) Date of filing: 12.01.2023
(51) Int. Cl.: A61M 16/00

(54) **"AN INSUFFLATOR FOR DETERMINING A VALUE OF A VOLUME OF INSUFFLATING GAS TO BE DRAWN FROM AN INSUFFLATED CAVITY DURING EVACTUATING OF THE CAVITY"**
INSUFFLATOR ZUR BESTIMMUNG EINES WERTS EINES INSUFFLATIONSGASVOLUMENS ZUM ZIEHEN AUS EINEM INSUFFLIERTEN HOHLRAUM WÄHREND DES EVAKUIERENS DES HOHLRAUMS
INSUFFLATEUR PERMETTANT DE DÉTERMINER UNE VALEUR D'UN VOLUME DE GAZ D'INSUFFLATION À ASPIRER D'UNE CAVITÉ AYANT FAIT L'OBJET D'UNE INSUFFLATION LORS D'UNE ÉVACUATION DE LA CAVITÉ

(30) Priority: 12.01.2022 IE S20220008
(43) Date of publication of application: 15.01.2025
(73) Proprietor: Palliare Limited, Dangan Galway (IE)
(72) Inventor: O'DEA, John, Galway (IE)
(74) Representative: Gorman, Francis Fergus
(86) International application number: PCT/IE2023/000002
(87) International publication number: WO 2023/135589

(56) References cited:
- WO-A1-2017/048429
- WO-A1-2017/122188
- WO-A1-2021/133688

## Description

The present invention relates to an insufflator for minimising the amount of residual insufflating gas remaining in a cavity in the body of a human or animal subject on completion of evacuating of the insufflating gas from the cavity, and preferably, for evacuating the insufflating gas from the cavity so that substantially no residual insufflating gas remains in the cavity on completion of evacuating of the insufflating gas from the cavity. The invention also relates to an insufflator configured for determining a value of a volume of insufflating gas to be drawn from an insufflated cavity in the body of a human or animal subject during evacuation of insufflating gas from the cavity, so that on completion of evacuation of the cavity, substantially no insufflating gas remains in the cavity. Further, the invention relates to an insufflator for insufflating a cavity in the body of a human or animal subject.

Medical insufflators are used to create a working volume in the body of a human or animal subject, in order to allow a surgeon to operate laparoscopically in the cavity. In particular, insufflators are used to create a pneumoperitoneum, namely, a working volume that allows a surgeon to operate laparoscopically in the peritoneal cavity of a human or animal subject, thereby providing a working volume therein for visualisation and manipulation of surgical tools and instruments during a minimally invasive surgical or investigative procedure, for example, a laparoscopic surgical procedure or a laparoscopic investigative procedure. During insufflating of the peritoneal cavity, insufflating gas is delivered to the cavity, and the cavity is maintained at a desired working pressure, sufficient to insufflate the cavity to provide a suitable working volume. During insufflating of the cavity, the cavity pressure in general is maintained at a working pressure in the range of 10mmHg to 30mmHg, and more typically, at a working pressure of approximately 15mmHg. On completion of the laparoscopic surgical or investigative procedure, insufflating gas is evacuated from the peritoneal cavity. However, it has been found that in many cases residual insufflating gas remains in the cavity when evacuation of the cavity has been completed and trocar accommodating openings in the wall of the peritoneal cavity have been sutured. This remaining residual insufflating gas may result in shoulder pain in the subject after completion of the laparoscopic surgical or investigative procedure, and in some cases, the shoulder pain my be quite intensive.

It has been shown that full evacuation of residual gas can significantly reduce post-operative pain ["Pulmonary recruitment can reduce residual pneumoperitoneum and shoulder pain in conventional laparoscopic procedures: results of a randomized controlled trial" by Garteiz-Martinez et al., Surgical Endoscopy (2021) 35:4143-4152].

WO2017/122188 describes an apparatus for insufflating a cavity in the body of a human or animal subject comprising a delivery means for delivering an insufflating fluid to a subject and a discharge control means for discharging fluid from the cavity.

There is therefore a need for an insufflator for minimising the amount of residual insufflating gas remaining in a cavity on completion of evacuating of the insufflating gas from the cavity, and preferably, for evacuating the insufflating gas from the cavity so that substantially no residual insufflating gas remains in the cavity on completion of evacuating of the insufflating gas from the cavity.

The present invention is directed towards providing such an insufflator, and the invention is also directed towards an insufflator configured for determining a value of a volume of insufflating gas to be drawn from an insufflated cavity in the body of a human or animal subject during evacuation of insufflating gas from the cavity, so that on completion of evacuation of the cavity, substantially no insufflating gas remains in the cavity. Further, the invention is directed towards an insufflator for insufflating a cavity in the body of a human or animal subject.

According to the invention there is provided an insufflator for insufflating a cavity in the body of a human or animal subject, the insufflator comprising:
a delivery means for delivering insufflating gas to the cavity for insufflating the cavity at a working pressure value,
a pressure sensor for monitoring pressure in the cavity and for producing a signal indicative of the pressure in the cavity,
a flow sensing device for monitoring flow of insufflating gas delivered to the cavity and for producing a signal indicative of the cumulative volume of insufflating gas delivered to the cavity from the commencement of delivery of the insufflating gas thereto or for producing a signal from which the cumulative volume of insufflating gas delivered to the cavity from the commencement of delivery of the insufflating gas thereto may be determined, and
a signal processor programmed:
   to operate the delivery means to deliver insufflating gas to the cavity for insufflating thereof, and
   to read signals from the pressure sensor and from the flow sensing device during insufflating of the cavity,
wherein the signal processor is programmed:
to read signals from the pressure sensor and from the flow sensing device from the commencement of insufflating of the cavity until the cavity pressure reaches a set pressure value, the set pressure value being less than the working pressure value,
to determine from the signals read from the pressure sensor and the flow sensing device from the commencement of insufflating of the cavity a value of an initial volume of insufflating gas delivered to the cavity, the initial volume of insufflating gas delivered to the cavity being the cumulative volume of insufflating gas delivered to the cavity from the commencement of delivery of the insufflating gas thereto until the cavity pressure reaches the set pressure value, and
to determine a value of a residual volume of insufflating gas to be drawn from the cavity during evacuating of insufflating gas from the cavity during termination of insufflating thereof, the value of the residual volume being determined as a function of the determined value of the initial volume of insufflating gas delivered to the cavity, so that during evacuating of insufflating gas from the cavity during termination of insufflating thereof, on the cavity pressure falling to the set pressure value, by evacuating a further volume of insufflating gas from the cavity substantially equal to the value of the residual volume thereof, substantially no insufflating gas remains in the cavity on completion of evacuating thereof.

In one embodiment of the invention the signal processor is programmed to determine the set pressure value.

In another embodiment of the invention the signal processor is programmed to determine a pressure/volume relationship between the pressure in the cavity and the cumulative volume of insufflating gas delivered to the cavity from the commencement of delivery thereof.

Preferably, the signal processor is programmed to compute the value of the increase in pressure in the cavity per unit volume of insufflating gas delivered to the cavity.

In another embodiment of the invention the signal processor is programmed to determine a first pressure/volume relationship and a subsequent second pressure/volume relationship from the determined pressure/volume relationship, the second pressure/volume relationship being different to the first pressure/volume relationship.

In another embodiment of the invention the signal processor is programmed to determine a part of the pressure/volume relationship during which the pressure in the cavity remains substantially constant as insufflating gas is being delivered to the cavity as the first pressure/volume relationship, and to determine a subsequent part of the pressure/volume relationship during which the pressure in the cavity increases linearly with respect to the delivery of insufflating gas to the cavity as the second pressure/volume relationship.

Preferably, the signal processor is programmed to determine the first and second pressure/volume relationships from the computed values of the increase in the pressure in the cavity per unit volume of insufflating gas delivered to the cavity.

In one embodiment of the invention the signal processor is programmed to determine a transition pressure value at which the first pressure/volume relationship transitions to the second pressure/volume relationship.

In another embodiment of the invention the signal processor is programmed to determine the transition pressure value as the value of the pressure in the cavity at the end of the first pressure/volume relationship.

In a further embodiment of the invention the signal processor is programmed to determine the transition pressure value as the value of the pressure in the cavity at the commencement of the second pressure/volume relationship.

In a further embodiment of the invention the signal processor is programmed to determine the transition pressure value as a pressure value between the value of the pressure in the cavity at the end of the first pressure/volume relationship and the pressure in the cavity at the commencement of the second pressure/volume relationship. Preferably, the signal processor is programmed to determine the transition pressure value as a pressure value substantially midway between the value of the pressure in the cavity at the end of the first pressure/volume relationship and the value of the pressure in the cavity at the commencement of the second pressure/volume relationship.

In one embodiment of the invention the signal processor is programmed to determine the set pressure value as a value not less than the determined transition pressure value.

In another embodiment of the invention the signal processor is programmed to determine the set pressure value at a pressure value equal to the determined transition pressure value or a value equal to the sum of the determined transition pressure value and a predefined pressure value. Preferably, the predefined pressure value lies in the range of 0.1mmHg to 3mmHg. Advantageously, the predefined pressure value lies in the range of 0.5mmHg to 2mmHg. Ideally, the predefined pressure value lies approximately equal to 1mmHg.

In one embodiment of the invention the signal processor is programmed to determine the value of the residual volume of insufflating gas as being substantially equal to the determined initial volume of the insufflating gas delivered to the cavity from the commencement of insufflating of the cavity until the pressure in the cavity reaches the set pressure value.

In another embodiment of the invention the signal processor is programmed to determine the value of the residual volume of insufflating gas as being equal to the determined initial volume of the insufflating gas delivered to the cavity from the commencement of insufflating until the pressure in the cavity reaches the set pressure value.

In a further embodiment of the invention the signal processor is programmed to determine the value of the residual volume of the insufflating gas as being a function of the value of the determined initial volume of insufflating gas delivered to the cavity from the commencement of insufflating thereof until the pressure in the cavity reaches the set pressure value and as a function of one or both of a compensating factor and a compensating constant. Preferably, the one or both of the compensating factor and the compensating constant are selected to compensate for one or both of any leakage of insufflating gas from the cavity during the insufflating of the cavity until the pressure in the cavity reaches the set pressure value and/or any leakage of air being drawn into the cavity during evacuating thereof. Advantageously, the compensating factor and/or the compensating constant are empirically derived.

In one embodiment of the invention the signal processor is programmed to read the values of the signals produced by the pressure sensor and the flow sensing device continuously or at predefined time intervals.

In another embodiment of the invention the signal processor is programmed to time-stamp, cross-reference and store in memory each pair of the values of the signals read from the pressure sensor and the flow sensing device.

In another embodiment of the invention the signal processor is programmed to compute the value of the cumulative volume of insufflating gas delivered to the cavity from the commencement of insufflating of the cavity and to determine the corresponding pressure in the cavity from each pair of the values of the signals read from the pressure sensor and the flow sensing device. Preferably, the signal processor is programmed to apply a smoothing algorithm in computing the cumulative volume of the insufflating gas delivered to the cavity, and advantageously, the smoothing algorithm comprises a moving average algorithm.

In one embodiment of the invention the signal processor is programmed to time-stamp, cross-reference and store in memory each computed value of the cumulative volume of insufflating gas delivered to the cavity and the corresponding pressure in the cavity.

Preferably, the signal processor is programmed to compute the value of the increase in pressure in the cavity per unit volume of insufflating gas delivered to the cavity and to determine the corresponding pressure in the cavity from each pair of the values of the signals read from the pressure sensor and the flow sensing device.

Advantageously, the signal processor is programmed to apply a smoothing algorithm in computing the value of the increase in pressure in the cavity per unit volume of insufflating gas delivered to the cavity, and preferably, the smoothing algorithm comprises a moving average algorithm.

in one embodiment of the invention the signal processor is programmed to time-stamp, cross-reference and store in memory each computed value of the increase in pressure in the cavity per unit volume of insufflating gas delivered to the cavity and the corresponding value of the pressure in the cavity,

In another embodiment of the invention the signal processor is programmed to determine the end of the first pressure/volume relationship from the computed values of the increase in pressure in the cavity per unit volume of insufflating gas delivered to the cavity,

In a further embodiment of the invention the signal processor is programmed to determine the end of the first pressure/volume relationship by comparing each computed value of the increase in pressure in the cavity per unit volume of insufflating gas delivered to the cavity with the previous or a number of the previously computed values of the increase in pressure in the cavity per unit volume of insufflating gas delivered to the cavity.

In another embodiment of the invention the signal processor is programmed to determine the commencement of the second pressure/volume relationship from the computed values of the increase in the pressure in the cavity per unit volume of insufflating gas delivered to the cavity.

In one embodiment of the invention the signal processor is programmed to determine the commencement of the second pressure/volume relationship by comparing each computed value of the increase in pressure in the cavity per unit volume of insufflating gas delivered to the cavity with the previous or a number of the previously computed values of the increase in pressure in the cavity per unit volume of insufflating gas delivered to the cavity.

In another embodiment of the invention the signal processor is programmed to determine a lower pressure value below which the set pressure value should not be set.

Preferably, the signal processor is programmed to determine the lower pressure value as being not less than or equal to the pressure in the cavity at the end of the first pressure/volume relationship.

In another embodiment of the invention the signal processor is programmed to determine an upper pressure value above which the set pressure value should not be set.

Preferably, the signal processor is programmed to determine the upper pressure value as being less than the working pressure value at which the insufflator is configured to operate during normal insufflating of the cavity.

In another embodiment of the invention the upper and lower pressure values are stored in memory.

Preferably, the delivery means is controlled by the signal processor to deliver the insufflating gas to the cavity at a relatively slow rate until the set pressure value has been determined.

Preferably, the signal processor is programmed to control the delivery means to deliver the insufflating gas to the cavity at a substantially constant pressure until the value of the initial volume of insufflating gas has been determined.

Advantageously, the signal processor is programmed to control the delivery means to deliver the insufflating gas to the cavity at a substantially constant rate until the value of the initial volume of the insuffiating gas has been determined.

Preferably, the signal processor is programmed to store the determined value of the set pressure in memory.

Advantageously, the signal processor is programmed to store the determined value of the residual volume of insufflating gas in memory.

In one embodiment of the invention the signal processor is programmed, on the value of the residual volume of the insufflating gas being determined, to control the delivery means to deliver the insufflating gas to the cavity at a pressure and a flow rate to insufflate the cavity to a working pressure value and to maintain the pressure in the cavity at the working pressure value.

In another embodiment of the invention the flow sensing device comprises a first flow sensor for monitoring the flow of insufflating gas delivered to the cavity.

In a further embodiment of the invention the insufflator further comprises an evacuating means for evacuating insufflating gas from the cavity, the signal processor being programmed to control the operation of the evacuating means for evacuating insuffiating gas from the cavity.

Preferably, the signal processor is programmed
to determine when the pressure in the cavity falls to the set pressure value from the signal read from the pressure sensor during evacuating of insufflating gas from the cavity,
to determine the cumulative volume of insufflating gas evacuated from the cavity from the time the pressure in the cavity falls to the set pressure value from the signal read from the flow sensing device, and
to terminate operation of the evacuating means on the determined cumulative volume of insufflating gas evacuated from the cavity from the time the pressure falls to the set pressure value being equal to the determined value of the residual volume of the insufflating gas.

In another embodiment of the invention the signal processor is programmed to read the values of the signals produced by the pressure sensor and the flow sensing device at the predefined time intervals during evacuating of the insufflating gas from the cavity.

In one embodiment of the invention the flow sensing device comprises a second flow sensor for monitoring the flow of insufflating gas evacuated from the cavity.

In another embodiment of the invention the second flow sensor comprises a flow rate sensor. Alternately, the second flow sensor comprises a flow metre.

In one embodiment of the invention the evacuating means comprises a vacuum pump.

In one embodiment of the invention the first flow sensor comprises a flow rate sensor. Alternately, the first Page flow sensor comprises a flow metre.

In one embodiment of the invention the set pressure value is selectable.

In another embodiment of the invention the working pressure value is selectable.

In one embodiment of the invention an interface means is provided for entering selectable values and data to the signal processor.

The advantages of the invention are many. A particularly important advantage of the invention is that at the end of evacuating of the insufflating gas from the cavity substantially no insufflating gas should remain in the cavity, and in many cases no insufflating gas remains in the cavity after evacuation of the cavity, thereby reducing post operative pain. This advantage is achieved by virtue of the fact that on the pressure in the cavity falling to the set pressure value during evacuating of insufflating gas from the cavity, a further volume of insufflating gas is drawn from the cavity, which is equal to the value of the residual volume of insufflating gas. Since the value of the residual volume of insufflating gas is equal to the cumulative volume of insufflating gas required to rise the cavity pressure to the set pressure value from commencement of insufflating thereof, therefore, during evacuating of insufflating gas from the cavity, when the pressure in the cavity falls to the set pressure value, the volume of insufflating gas remaining in the cavity must be approximately equal to the value of the residual volume of insufflating gas. Accordingly, by evacuating the further volume of insufflating gas from the cavity on the pressure therein falling to the set pressure value, which is substantially equal to the value of the residual volume of insufflating gas, substantially all, if not all of the insufflating gas should be withdrawn from the cavity at the end of evacuating the insufflating gas from the cavity.

The invention will be more clearly understood from the following description of some preferred embodiments thereof, which are given by way of example only with reference to the accompanying drawings, in which:
Fig. 1 is a block representation of an insufflator according to the invention,
Fig. 2 is a graphical representation of a pressure/volume relationship between pressure in the cavity in the body of a human or animal subject being insufflated by the insufflator, and the cumulative volume of the insufflating gas delivered to the cavity, with volume plotted on the abscissa and pressure plotted on the ordinate, and
Fig. 3 is an enlarged view of a portion of the graphical representation of the pressure/volume relationship of Fig. 2.

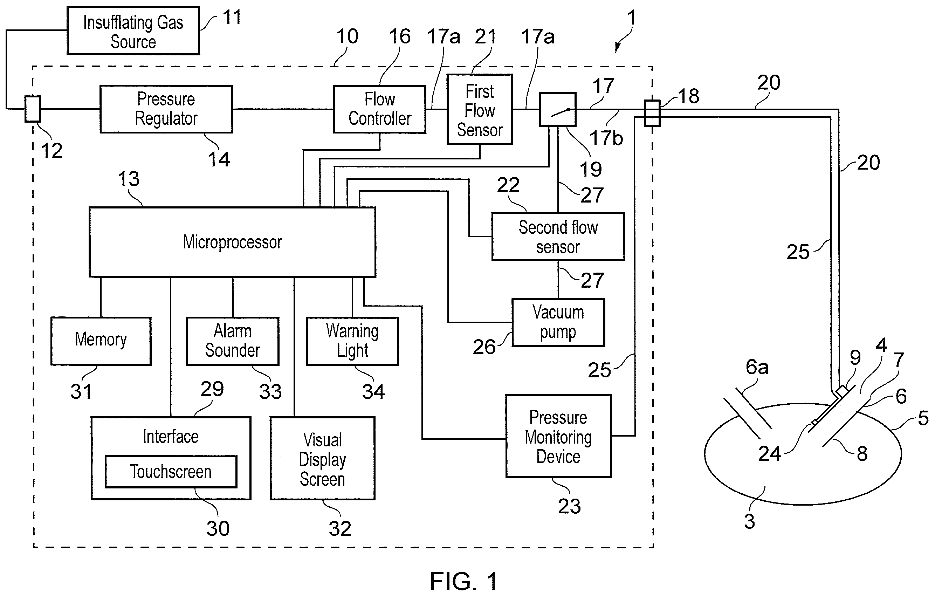

Referring to the drawings and initially to Fig. 1 thereof there is illustrated an insufflator according to the invention indicated generally by the reference numeral 1 for insufflating a cavity, in this embodiment of the invention the peritoneal cavity 3 in the body 5 of a human or animal subject, during a minimally invasive surgical or investigative procedure being carried out, typically, laparoscopically. Although, it will be readily apparent to those skilled in the art that the insufflator 1 may be used for insufflating any lumen, vessel or cavity in the body of a human or animal subject, laparoscopically, endoscopically or otherwise. The insufflator 1, as will be described in more detail below, is configured to insufflate the cavity 3 through a trocar 6 inserted into the cavity 3, to maintain the insufflated cavity at a desired selectable working pressure during the carrying out of the procedure, and on completion of the procedure to evacuate the insufflating gas from the cavity 3, so that on completion of evacuating of the cavity 3 substantially no insufflating gas remains in the cavity 3, as will be described in detail below.

The trocar 6 comprises an instrument channel 4 extending therethrough, and extends from a proximal end 7 to a distal end 8, which is located in the cavity 3. The trocar 6 may or may not comprise an insufflating gas inlet port 9 to which insufflating gas is delivered to the trocar 6. A bore (not shown) extending along the wall of the trocar 6 from the inlet port 9 to the distal end 8 of the trocar 6 accommodates insufflating gas from the inlet port 9 into the cavity 3.

The insufflator 1 comprises a housing 10, which may include a source of insufflating gas, which if included in the housing 10 would normally be provided in a pressurised container containing pressurised insufflating gas, typically, carbon dioxide. However, in this embodiment of the invention the insufflator 1 is adapted to receive the pressurised insufflating gas from an external source 11, which typically, comprises compressed carbon dioxide, typically, from a source of compressed carbon dioxide available in a hospital operating theatre, or elsewhere where the insufflator 1 is being used. An inlet port 12 is provided in the housing 10 for connecting the insufflator 1 to the external pressurised source 11 of the insufflating gas. A signal processor, in this case provided by a microprocessor 13 located in the housing 10 controls the operation of the insufflator 1 as will be described below. However, it will be appreciated that any other suitable form of signal processor may be provided, for example, a microcontroller or other such suitable signal processor.

A pressure regulator 14 located in the housing 10 is connected to the inlet port 12 for receiving the insufflating gas therefrom, and for stepping down the pressure of the insufflating gas to a pressure in the order of 40mmHg.

A delivery means comprising a flow controller 16 located in the housing 10 controls the flow rate of the insufflating gas to the cavity 3 of the subject, and in turn the pressure in the cavity 3. The flow controller 16 is connected to the pressure regulator 14 and receives the insufflating gas from the pressure regulator 14 at the stepped down pressure. The flow controller 16 is operated under the control of the microprocessor 13 for controlling the supply of the insufflating gas and the flow rate thereof at which the insufflating gas is delivered to the cavity 3 of the subject for insufflating the cavity 3, and for controlling the pressure in the cavity 3, as will be described below.

A connecting tube 17 connects the flow controller 16 to an outlet port 18 of the housing 10 through a solenoid operated two-way valve 19 also located in the housing 10 and described below. The connecting tube 17 comprises a first part 17a, which connects the flow controller 16 to the two-way valve 19, and a second part 17b, which connects the two-way valve 19 to the outlet port 18.

A gas line 20 from the outlet port 18 supplies the insufflating gas to the cavity 3 of the subject through the trocar 6. The gas line 20 may be entered directly into the cavity 3 through the instrument channel 4 in the trocar 6, or if the trocar 6 is provided with the gas inlet port 9, the gas line 20 may be connected to the gas inlet port 9, through which the insufflating gas is delivered into the cavity 3.

A flow sensing device comprising a first flow rate sensor 21 and a second flow rate sensor 22 are located in the housing 10. The second flow rate sensor 22 and its function will be described in detail below. The first flow rate sensor 21 is located in the first part 17a of the connecting tube 17, and monitors the flow rate of the insufflating gas delivered through the first part 17a of the connecting tube 17 to the cavity 3, and produces a signal indicative of the rate at which the insufflating gas is being delivered to the cavity 3. The microprocessor 13 is programmed to read the value of the signal produced by the first flow rate sensor 21 and to compute the cumulative volume of the insufflating gas delivered to the cavity 3 from the commencement of delivery of insufflating gas thereto.

A pressure monitoring device 23 located in the housing 10 monitors cavity pressure, namely, the pressure in the cavity 3 of the subject and produces a signal indicative of the pressure in the cavity 3. The pressure monitoring device 23 reads signals from a pressure sensor 24 which may be located in the housing 10 in the connecting tube 17 downstream of the first flow rate sensor 21, or may be located in the cavity 3, for example, on a portion of the trocar 6 located within the cavity 3, which would give a direct reading of the cavity pressure. If the pressure sensor were located in the connecting tube 17 downstream of the first flow rate sensor 21, during monitoring of the cavity pressure, in order to determine the true pressure in the cavity 3, either the flow controller 16 would isolate the cavity 3 from the insufflating gas during measuring of the pressure by the pressure sensor in order to give a true value of the pressure in the cavity 3, or alternatively, the pressure of the insufflating gas flowing through the connecting tube 17 would be read from the pressure sensor. The pressure monitoring device 23 would then apply a compensating factor to the pressure value read from the pressure sensor to obtain the true value of the pressure in the cavity 3.

However, in order to determine the value of the compensating value to be applied to the signals indicative of the pressure of the insufflating gas flowing in the connecting tube 17, the pressure monitoring device 23 would obtain the flow rate of the insufflating gas flowing in the connecting tube 17 from either the first flow rate sensor 21 or the microprocessor 13, and would apply a suitable compensating factor to the signal read from the pressure sensor taking account of the read pressure value, the flow rate of the insufflating gas in the connecting tube 17, the frictional resistance to flow in the connecting tube 17 and in the gas line 20 between the pressure sensor and the trocar 6.

Alternatively, the pressure sensor, if located in the housing 10 may be connected directly to the cavity 3 through a pressure monitoring conduit (not shown) which would extend from the pressure sensor through the trocar 6 into the cavity 3 or through a second trocar 6a, similar to the trocar 6 extending into the cavity 3, so that the pressure monitored by the pressure sensor would be the true pressure in the cavity 3.

In this embodiment of the invention the pressure sensor 24 is located on an outer surface of the trocar 6 adjacent the distal end 8 thereof, and produces a signal indicative of the pressure in the cavity 3. An electrically conductive wire 25 connects the pressure sensor 24 to the pressure monitoring device 23 to apply the signal from the pressure sensor 24 to the pressure monitoring device 23. The wire 25 extends along and is secured to the outer surface of the gas line 20 and enters the housing 10 with the gas line 20 at the outlet port 18, and then extends to and is connected to the pressure monitoring device 23. However, it is envisaged that in some embodiments of the invention the communication between the pressure sensor 24 and the pressure monitoring device 23 may be provided wirelessly.

The microprocessor 13 is programmed to read the value of the signal from the pressure monitoring device 23, which are indicative of the pressure in the cavity 3, and to control the flow controller 16 in response to the value of the signal read from the pressure monitoring device 23 for controlling the supply and the flow rate of the insufflating gas to the cavity 3, for in turn maintaining the pressure in the cavity at a selected working pressure in the range of 10mmHg to 15mmHg during insufflating of the cavity 3,

An evacuating means, in this embodiment of the invention a vacuum pump 26 is located in the housing 10 and is operated under the control of the microprocessor 13 for evacuating insufflating gas from the cavity 3 on completion of the surgical or investigative procedure, as will be described below. A vacuum line 27 extending from the vacuum pump 26 is connected to the connecting tube 17 through the two-way valve 19. The two-way valve 19 is selectively and alternatively operated under the control of the microprocessor 13 in a first insufflating mode connecting the second part 17b of the connecting tube 17 to the first part 17a thereof during insufflating of the cavity 3, and in a second evacuating mode connecting the second part 17b of the connecting tube 17 during evacuating of insufflating gas from the cavity 3. When the two-way valve is in the first insufflating mode the vacuum line 27 is isolated from the connecting tube 17, and when the two-way valve is in the second evacuating mode the first part 17a of the connecting tube 17 is isolated from the second part 17b thereof. The second flow rate sensor 22 is located in the vacuum line 27 and monitors the flow rate of insufflating gas being drawn from the cavity 3 through the vacuum line 27 by the vacuum pump 26 and produces a signal indicative of the flow rate at which insufflating gas is being drawn from the cavity 3. The signal processor 13 is programmed to read the value of the signal produced by the second flow rate sensor 22, and to control the operation of the vacuum pump 26 in response to the value of the signal read from the second flow rate sensor 22, as will be described below.

An interface 29 which may comprise a touchscreen or a keypad, in this case a touchscreen 30 is provided on the housing 10 to enable entry to the microprocessor 13 of a desired selectable working pressure value to which the cavity 3 is to be insufflated and at which the pressure is to be maintained in the cavity 3 during insufflating of the cavity 3 during the surgical or investigative procedure. The touchscreen 30 is also configured for inputting other relevant data, for example, a maximum safe pressure to which the cavity 3 may be insufflated, and other relevant data, which will be well known to those skilled in the art. Additionally, the touch screen 30 is configured for inputting other data to the microprocessor 13 which may be required by the microprocessor 13 during both insufflating and evacuating the cavity 3 as will be described below,

A memory 31 of the microprocessor 13 which may be any suitable electronic memory, such as a random access memory, is provided for storing data for access by the microprocessor 13, as will also be described below. The microprocessor 13 controls a visual display screen 32, which provides visual data to a surgeon and/or a clinician in an operating theatre. An alarm sounder 33 for producing an audible alarm signal, and a warning light 34 for producing a visual warning signal, are also operated under the control of the microprocessor 13. Typically, the alarm sounder 33 and the warning light 34 are operated under the control of the microprocessor 13 to sound an alarm and to produce a visual light signal in response to the cavity pressure exceeding the maximum safe pressure. The alarm sounder 33 and the warning light 34 typically are mounted on the housing 10, but may be located at any suitable location to be heard and seen by the surgeon and/or the clinician participating in the carrying out of the procedure.

With the insufflator 1 connected to the external insufflating gas source 11 through the inlet port 9, and with the gas line 20 connected to the trocar 6, and the wire 25 connecting the pressure sensor 24 to the pressure monitoring device 23, the insufflator 1 is ready for use.

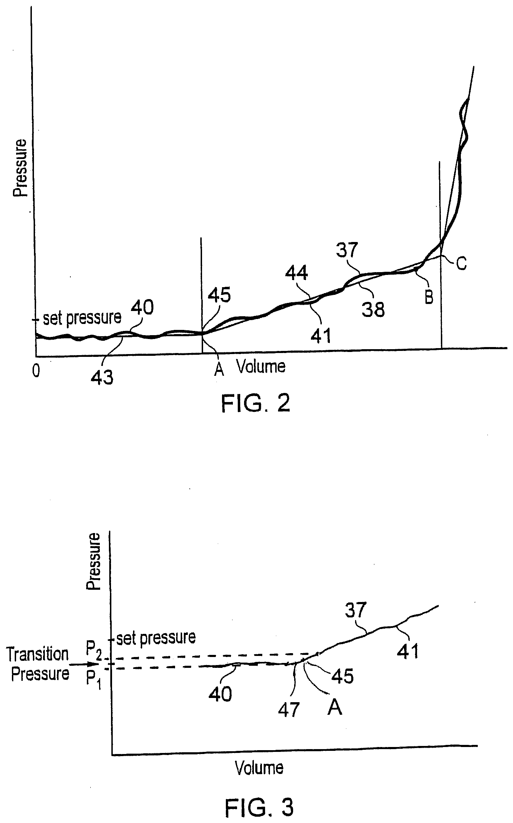

Before describing the operation of the insufflator 1, reference will now be made to Fig. 2. Fig. 2 illustrates two graphs, namely, a first graph line 37 and a second graph line 38 of the pressure in the cavity 3 plotted against the cumulative volume of insufflating gas delivered to the cavity 3 from the commencement of delivery of the insufflating gas to the cavity 3. In Fig. 2, the pressure in the cavity 3 is plotted on the ordinate and the cumulative volume of insufflating gas in the cavity 3 is plotted on the abscissa. The first graph line 37 represents a plot of the actual pressure in the cavity 3 plotted against the actual cumulative volume of insufflating gas delivered to the cavity 3 from the commencement of delivery of insufflating gas to the cavity 3, and represents the actual pressure/volume relationship between the pressure in the cavity 3 and the cumulative volume of insufflating gas delivered in the cavity 3. The second graph line 38 represents a smoothed version of the plot of the first graph line 37, and represents a smoothed version of the pressure/volume relationship between the pressure in the cavity 3 and the cumulative volume of insufflating gas delivered to the cavity 3.

A first part 40 of the first line 37, from the commencement of delivery of insufflating gas to the cavity 3 to point A of the first line 37, represents a first pressure/volume relationship of the pressure/volume relationship between the pressure in the cavity 3 and the cumulative volume of insufflating gas delivered to the cavity 3. A second part 41 of the line 37, from the point A to the point B on the line 37, represents a second pressure/volume relationship of the pressure/volume relationship between the pressure in the cavity 3 and the cumulative volume of insufflating gas delivered to the cavity 3.

A first part 43 of the second line 38, from the commencement of delivery of insufflating gas to the cavity 3 to the point A on the second line 38, represents a smoothed version of the first pressure/volume relationship of the pressure/volume relationship between the pressure in the cavity 3 and the cumulative volume of insufflating gas delivered to the cavity 3. A second part 44 of the second line 38, from the point A to the point C thereof, represents a smoothed version of the first pressure/volume relationship of the pressure/volume relationship between the pressure in the cavity 3 and the cumulative volume of insufflating gas delivered to the cavity 3. In both the first and second lines 37 and 38, the first pressure/volume relationship transitions to the second pressure/volume relationship at a point of inflection 45 at the point A on the first and second lines 37 and 38.

During the first pressure/volume relationship to the point of inflection A on the first and second lines 37 and 38, the cavity pressure remains substantially constant at a few millimetres of mercury (mmHg) typically up to 6mmHg, while the insufflating gas is continuously delivered to the cavity 3 to fill the cavity 3. However, during the second pressure/volume relationship from the point of inflection A onwards to the point B on the first line 37 and to the point C on the second line 38, the pressure commences to rise as the insufflating gas continues to be delivered to the cavity 3. As can be seen from the first part 40 of the first line 37 and from the first part 43 of the second line 38, the increase in pressure in the cavity 3 per unit volume of insufflating gas delivered to the cavity 3 remains substantially constant at a value of zero. In other words, the slope of the first part 40 of the first line 37 and the first part 43 of the second line 38 is approximately of value zero. As can be seen from the second part 41 of the first line 37 and from the second part 44 of the second line 38, the second pressure/volume relationship is a substantially linear pressure/volume relationship with the pressure in the cavity 3 increasing with respect to the cumulative volume of insufflating gas delivered to the cavity 3. In other words, the increase in pressure in the cavity 3 per unit volume of insufflating gas delivered to the cavity 3, which is the slope of the second parts 41 and 44 of the first and second lines 37 and 38, remains substantially constant at a value greater than zero. The working pressure in the cavity 3 is selected to be a pressure lying on the second parts 41 and 44 of the first and second lines 37 and 38, in other words, a pressure of value between the pressure at the point of inflection 45 to the point B on the first line 37 or to the point C on the second line 38, and in general, a pressure closer to the pressure at the point B on the first line 37 or to the point C on the second line 38, and as discussed above, the working pressure, in general, is selected to lie in the range of 10mmHg to 15mmHg. From the point B on the first line 37 and the point C on the second line 38, the increase in pressure in the cavity 3 per unit volume of insufflating gas delivered to the cavity increases significantly due to the reduction in compliance of the cavity 3 as the cavity expands. Therefore, from the points B and C on the first and second lines 37 and 38, respectively, there is little gain in working volume in the cavity 3 for each unit increase in the pressure in the cavity 3.

It is believed that the reason in conventional insufflators, that some insufflating gas remains in a cavity after evacuating of the cavity is as follows. On commencement of insufflating of the cavity 3, a volume of insufflating gas must be delivered to the cavity before the pressure in the cavity 3 commences to rise, in other words, the cumulative volume of insufflating gas delivered to the cavity 3 during the first pressure/volume relationship. In general, during normal evacuating of insufflating gas from a cavity of a subject, the insufflator is operated to continue evacuating insufflating gas from the cavity while the pressure in the cavity 3 continues to fall. However, once the cavity pressure ceases to fall, evacuating of the cavity is terminated. If one considers the first and second lines 37 and 38 of Fig. 2, as being the reverse of the pressure/volume relationship between the pressure in the cavity 3 and the cumulative volume of insufflating gas remaining in the cavity during evacuating of insufflating gas from of the cavity 3, then the pressure in the cavity 3 during evacuating thereof would cease to fall further from the point of inflection 45 onwards. Therefore, by terminating evacuating of the cavity 3 at the pressure at the point of inflection 45, a volume of insufflating gas remains in the cavity which is not evacuated from the cavity 3. Accordingly, it is not possible to determine when evacuation of the cavity should be terminated in order to remove substantially all the insufflating gas from the cavity.

The method according to the invention is directed towards addressing this problem.

The invention addresses the problem by determining a set pressure value during the initial insufflating of the cavity 3 to the selected working pressure. The set pressure value is not less than the pressure in the cavity at the point of inflection 45 on the first and second lines 37 and 38, and preferably, is slightly greater than the pressure in the cavity at the point of inflection 45. In other words, the set pressure value is set at a value not less than the value of the transition pressure in the cavity 3 at which the first pressure/volume relationship transitions to the second pressure/volume relationship, and preferably, at a value slightly greater than the value of the transition pressure,

On the set pressure value having been determined, the initial volume of insufflating gas delivered to the cavity from the commencement of insufflating of the cavity until the pressure in the cavity 3 reaches the set pressure value is computed. The initial volume of insufflating gas delivered to the cavity 3 is determined by computing the cumulative volume of insufflating gas delivered to the cavity from the commencement of insufflating the cavity until the pressure in the cavity reaches the set pressure value. The initial volume of insufflating gas delivered to the cavity 3 is deemed to be a residual volume of insufflating gas. The residual volume of insufflating gas is the volume of insufflating gas which must be withdrawn from the cavity 3 during evacuating of insufflating gas therefrom from the time the pressure in the cavity has fallen to the set pressure value, so that substantially no insufflating gas should remain in the cavity 3 on completion of evacuating of the insufflating gas from the cavity 3,

The set pressure value may be any pressure value along the second part 41 and the second part 44 of the first and second lines 37 and 38, respectively, and in general, is set at a pressure closer to the transition pressure in the cavity 3 at the point of inflection 45, rather than at the pressure at the points B or C on the first and second lines 37 and 38, Preferably, the set pressure value is computed as the sum of the value of the transition pressure in the cavity 3 at the point A and a predefined pressure value. The predefined pressure value, typically, lies in the range of 0.1mmHg to 3mmHg, and in general, the predefined pressure value is approximately 1mmHg.

Once the set pressure value and the value of the residual volume of the insufflating gas have been determined, the set pressure value and the value of the residual volume of insufflating gas are stored in the memory 31 of the microprocessor 13. Thus, on completion of the procedure during evacuating of the cavity 3, the pressure in the cavity 3, as will be described below, is monitored, and on the pressure in the cavity 3 falling to the set pressure value, the cumulative volume of insufflating gas drawn from the cavity from the time the pressure in the cavity 3 falls to the set pressure value is monitored. On the cumulative volume of insufflating gas drawn from the cavity from the time the pressure in the cavity 3 falls to the set pressure value being equal to the value of the residual volume of insufflating gas, operation of the vacuum pump 26 is terminated,

The set pressure value may be selected, and inputted through the touchscreen 30. However, in this embodiment of the invention the microprocessor 13 is programmed to determine the set pressure value during initial insufflating of the cavity 3, as will be described below.

Turning now to the determining of the set pressure value, the microprocessor 13 is programmed during initial insufflating of the cavity 3 to deliver the insufflating gas to the cavity at a constant flow rate, typically, at a rate in the range of 0.5 litres per minute to 5 litres per minute, and more typically at a rate in the range of 0.5 litres per minute to 3 litres per minute. The microprocessor 13 reads the values of the signals produced by the pressure monitoring device 23 and the first flow rate sensor 21 at predefined sampling intervals, which in this embodiment of the invention are predefined time intervals of 10 milliseconds. As the values are successively read of the signals produced by the pressure monitoring device 23 and the first flow rate sensor 21, the corresponding values of each pair thereof are time stamped, cross-referenced and stored in the memory 31. As each pair of values are read of the signals produced by the pressure monitoring device 23 and the first flow rate sensor 21, the microprocessor 13 is programmed to compute the current cumulative volume of insufflating gas delivered to the cavity from the commencement of insufflating thereof from the value of the signal read from the first flow rate sensor 21 and the elapsed time since the commencement of insufflating of the cavity, and the computed cumulative volume of insufflating gas delivered to the cavity is time-stamped and cross-referenced with the corresponding value of pressure in the cavity 3 and stored in the memory 31.

As the cumulative volumes of insufflating gas delivered to the cavity 3 and the corresponding values of the pressure in the cavity 3 are computed and determined, the microprocessor 13 is programmed to compute a pressure/volume relationship between the pressure in the cavity 3 and the cumulative volume of insufflating gas delivered to the cavity 3, which is stored in the memory 31 in a machine readable form, for example, in a tabular form. From the determined pressure/volume relationship, the first pressure/volume relationship corresponding to the first part 40 of the first line 37 of Fig. 2 is determined, and the second pressure/volume relationship corresponding to the second part 41 of the first line 37 of Fig. 2 is likewise determined. The transition pressure is then determined as being the pressure in the cavity at which the first pressure/volume relationship transitions to the second pressure/volume relationship, in other words, the pressure in the cavity at the end of the first pressure/volume relationship, which in general will be the constant pressure in the cavity 3 during the initial period while insufflating gas is continuously delivered to the cavity without an increase in pressure in the cavity. However, in the event that the slope of the first part 40 of the first line 37 is not zero and rises gradually to the transition pressure, the transition pressure is determined as being the pressure in the cavity at the end of the first pressure/volume relationship.

During computation of each value of the cumulative volume of insufflating gas delivered to the cavity 3 from the commencement of insufflating thereof, the microprocessor is programmed to apply a smoothing algorithm, which in this embodiment of the invention comprises a moving average algorithm to the computation in order to produce a smoothed version of the pressure/volume relationship between the pressure in the cavity 3 and the corresponding cumulative volume of insufflating gas delivered to the cavity as represented by the second line 38 of Fig. 2, The microprocessor 13 is programmed to determine the transition pressure from the smoothed values of the pressure/volume relationship.

With the transition pressure having been determined, the microprocessor 13 is programmed to compute the value of the set pressure by adding the predefined pressure value of 1mmHg to the determined value of the transition pressure.

The microprocessor 13 is then programmed to determine the initial volume of insufflating gas delivered to the cavity 3 from the commencement of insufflating thereof to the time the pressure in the cavity 3 reaches the set pressure value by reading the cumulative volume of insufflating gas delivered to the cavity from the commencement of insufflating thereof until the pressure in the cavity 3 reached the set pressure value from the cross-referenced values of the cumulative volume of insufflating gas delivered to the cavity 3 and the corresponding values of the pressure in the cavity 3. The value of the residual volume of insufflating gas is then determined by the microprocessor 13 as being equal to the initial volume of insufflating gas delivered to the cavity 3 from the commencement of insufflating thereof until the pressure in the cavity has reached the set pressure value. If however leakage of insufflating gas is occurring during the determining and computation of the value of the residual volume of insufflating gas, the value of the residual volume of insufflating gas may be computed as a function of the initial volume of insufflating gas multiplied by a compensating factor, to compensate for the leakage of insufflating gas, or the value of the residual volume of insufflating gas may be determined as the sum of the initial volume of insufflating gas and a compensating constant. The compensating factor or the compensating constant would be determined empirically.

Once the set pressure value has been determined and the value of the residual volume of the insufflating gas has been determined, the set pressure value and the value of the residual volume of insufflating gas are stored in the memory 31.

Alternatively, or additionally, the microprocessor 13 may be programmed to compute the increase in the pressure in the cavity 3 per unit volume of insufflating gas delivered to the cavity each time the values of the signals are read from the pressure monitoring device 23 and from the first flow rate sensor 21. Each computed value of the increase in pressure in the cavity per unit volume of insufflating gas delivered to the cavity is time-stamped and cross-referenced with the corresponding value of the pressure in the cavity 3, and the cross-referenced values of the increase in pressure in the cavity per unit volume of insufflating gas delivered to the cavity and the corresponding value of the pressure in the cavity 3 are cross-referenced and stored in the memory 31. In computing each value of the increase in pressure in the cavity 3 per unit volume of insufflating gas delivered to the cavity, a moving average algorithm is applied to the computation in order to smooth the computed values.

Since the increase in pressure in the cavity per unit volume of insufflating gas delivered to the cavity is represented by the slope of the first and second lines 37 and 38 of Fig. 2, the microprocessor 13 is programmed to determine the transition pressure in the cavity 3 as being the pressure in the cavity 3 when the first pressure/volume relationship represented by the first part 43 of the second line 38 transitions to the second pressure/volume relationship represented by the second part 44 of the second line 38, in other words, when the slope of the pressure/volume relationship of the second line 38 transitions from being approximately zero to a value greater than zero.

In some embodiment of the invention the pressure/volume relationship between the pressure in the cavity 3 and the cumulative volume of insufflating gas delivered to the cavity may transition from the first pressure/volume relationship to the second pressure/volume relationship through an intermediate pressure/volume relationship, in which the pressure/volume relationship is no longer linear, but in which the pressure/volume relationship commences to increase. In Fig. 3 such an intermediate pressure/volume relationship is represented by an intermediate portion 47 of the enlarged portion of the first graph line 37 adjacent the point A. In cases where the first pressure/volume relationship transitions to the second pressure/volume relationship through a non-linear intermediate pressure/volume relationship, the microprocessor 13 is programmed to determine a first pressure value P₁ and a second pressure value P₂ as illustrated in Fig. 3. The microprocessor 13 is programmed to determine the first pressure value P₁ as being the pressure in the cavity 3 at the end of the first pressure/volume relationship, and the microprocessor 13 is programmed to determine the second pressure value P₂ as the pressure in the cavity 3 at the commencement of the second pressure/volume relationship. The microprocessor 13 is programmed to compute the transition pressure value as being equal to the average value of the determined first and second pressure values P₁ and P₂.

The set pressure value is then computed from the determined transition pressure value by adding the predefined pressure value of 1mmHg to the value of the computed transition pressure. With the set pressure value thus computed, the set pressure value is stored in the memory 31. The value of the residual volume of the insufflating gas is determined as being the cumulative volume of insufflating gas delivered to the cavity 3 from the commencement of insufflating thereof until the pressure in the cavity reaches the determined set pressure value, which is determined from the pressure/volume relationship stored in the memory 31.

Alternatively, if there is leakage of insufflating gas from the cavity during insufflating thereof, the value of the residual volume of insufflating gas may be determined as a function of the cumulative volume of insufflating gas delivered to the cavity from the commencement of insufflating thereof until the pressure in the cavity reaches the set pressure value multiplied by a compensating factor, or added to a compensating constant as already described.

With the set pressure value determined and stored in the memory 31 and the value of the residual volume of insufflating gas computed and stored in the memory 31, the microprocessor 13 controls the flow controller 16 to increase the rate at which the insufflating gas is delivered to the cavity 3 until the pressure in the cavity 3 reaches the selected working pressure. The microprocessor 13 then continues to control the flow controller 16 for maintaining the pressure in the cavity 3 at the selected working pressure.

On completion of the procedure in the cavity, the microprocessor 13 is programmed to terminate operation of the flow controller 16 in order to terminate delivery of insufflating gas to the cavity 3, and to operate the two-way valve 19 to connect the second part 17b of the connecting tube 17 to the vacuum line 27. The microprocessor 13 then activates the vacuum pump 26 to evacuate insufflating gas from the cavity 3. During operation of the vacuum pump 26, the microprocessor 13 reads signals from the pressure monitoring device 23 at the predefined time intervals of 10 milliseconds. When the pressure in the cavity 3 falls to the set pressure value, the microprocessor 13 commences to read the values of the signal produced by the second flow rate sensor 22 at the predefined time intervals of 10 milliseconds. As each value of the signal produced by the second flow rate sensor 22 is read, the microprocessor 13 is programmed to compute the cumulative volume of insufflating gas drawn from the cavity 3 by the vacuum pump 26 from the time the pressure in the cavity 3 fell to the set pressure value. The cumulative volume of the insufflating fluid drawn from the cavity 3 is computed from the current value of the signal read from the second flow rate sensor 22 and the time elapsed since the pressure in the cavity fell to the set pressure value. As each value of the cumulative volume of the insufflating gas drawn from the cavity 3 from the time the cavity pressure fell to the set pressure value, the just computed value of the cumulative volume is compared with the value of the residual volume of insufflating gas stored in the memory 31.

The microprocessor 13 is programmed to immediately terminate operation of the vacuum pump 26 when the cumulative volume of insufflating gas drawn from the cavity 3 by the vacuum pump 26 from the time the pressure in the cavity 3 fell to the set pressure value, is equal to the value of the residual volume of insufflated gas stored in the memory 31.

Thereby, by drawing a volume of insufflating gas from the cavity 3 from the time the pressure in the cavity 3 falls to the set pressure value, which is equal to the stored value of the residual volume of insufflating gas, substantially no insufflating gas should remain in the cavity on termination of the operation of the vacuum pump 26.

In an alternative embodiment of the invention the second flow rate sensor 22 may be omitted from the vacuum line 27, and in which case, the vacuum pump 26 would be a constant volume vacuum pump. In this embodiment of the invention during evacuation of the cavity 3 on completion of the procedure, when the pressure in the cavity 3 falls to the set pressure value, the vacuum pump would be operated for a time period equivalent to the time period required to withdraw a volume of insufflating gas from the cavity 3 equal to the value of the residual value of insufflating gas stored in the memory 31. At the end of the time period, the vacuum pump would be deactivated, since at that stage substantially no insufflating gas should remain in the cavity 3.

It is also envisaged that instead of the insufflating and evacuation of the cavity being carried out through the same gas line, namely, the gas line 20, a separate evacuation line may be provided from the vacuum pump to the cavity 3. In which case, the evacuating line would be connected through an inlet port in the housing to the vacuum pump, and would be inserted into the cavity 3 through, for example, the second trocar 6a, or through a Veress needle or other suitable access device. Depending on the vacuum pump, whether it be a constant volume vacuum pump or otherwise, the evacuating line would be connected to the vacuum pump either directly or through the second flow rate sensor 22. The provision of a separate evacuating line would eliminate the need for the two-way valve 19.

In this embodiment of the invention due to the fact that insufflating gas may leak from the cavity 3 from the commencement of insufflating thereof up to the time the cavity pressure reaches the set pressure value, and also since air will leak into the cavity during evacuating of the cavity from and after the time the pressure in the cavity has fallen to the set pressure value, the residual pressure value is computed to take account of both such leakages of insufflating gas from the cavity and air into the cavity. While complex computations can be carried out to very precisely determine the amount of insufflating gas which leaked from the cavity during inflating thereof up to the time the cavity pressure reaches the set pressure value, and for determining the leakage of air into the cavity during evacuating thereof from the time the pressure in the cavity has fallen to the set pressure value, in this embodiment of the invention, a more simple method of determining both leakage from and leakage into the cavity is used. The leakage of insufflating gas from the cavity during insufflating thereof up to the time the cavity pressure reaches the set pressure value, is determined by determining the rate of leakage of insufflating gas from the cavity immediately upon the cavity having been insufflated to the working pressure and being maintained at the working pressure for a short period thereafter. The rate of leakage of insufflating gas to maintain the cavity insufflated at the working pressure is determined as the rate at which insufflating gas is being delivered to the cavity to maintain the pressure in the cavity at the working pressure. The total leakage of insufflating gas from the commencement of insufflating of the cavity up to the time the pressure in the cavity reaches the set pressure value is then determined by multiplying the determined rate of leakage of insufflating gas from the cavity by the time period from the commencement of insufflating of the cavity up to the time the cavity pressure reaches the set pressure value. This computed total value of the insufflating gas leaked from the cavity is then subtracted from the already computed initial volume of insufflating gas, which is equal to the cumulative volume of insufflating gas delivered to the cavity up to the time the cavity pressure reached the set pressure value.

This corrected initial value of the volume of insufflating gas is then further corrected to take account of leakage of air into the cavity during evacuating of the cavity from the time the cavity pressure falls to the set pressure value. The rate of leakage of air into the cavity during the evacuating thereof is determined as being equal to the leakage of insufflating gas from the cavity while the cavity pressure is being maintained at the working pressure for a short time just prior to termination of insufflating of the cavity. This rate of leakage of insufflating gas from the cavity is deemed to be substantially equal to the rate at which air would leak into the cavity during evacuating thereof, and the total volume of air expected to be drawn into the cavity during evacuating thereof from the time the pressure in the cavity falls to the set pressure value is determined by multiplying the determined rate of leakage of air into the cavity by the expected time to evacuate the value of the residual volume of insufflating gas from the cavity when corrected. The already corrected initial volume of insufflating gas is further corrected by adding the total volume of air expected to leak into the cavity during evacuating thereof from the time the pressure in the cavity falls to the set pressure value.

For example, if

| | |
|---|---|
| the total cumulative volume of insufflating gas delivered to the cavity on the commencement of delivery of insufflating gas to the cavity up to the time the cavity pressure reached the set pressure value | = V₁ litres |
| the rate of leakage of insufflating gas from the cavity | = L₁ litres/minute |
| and the time taken to insufflate the cavity from the commencement of insufflating until the pressure in the cavity reached the set pressure value | = t₁ minutes |
| then the total value of insufflating gas which leaked from the cavity from the commencement of inflating thereof to the time the cavity pressure reached the set pressure value | = L₁.t₁ litres |
| therefore, the corrected value of the initial volume of insufflating gas delivered to the cavity from the commencement of insufflating thereof to the time the cavity pressure reached the set pressure value | = V₁ - L₁.t₁ litres |
| if it is estimated that the time to evacuate the cavity from the time the cavity pressure falls to the set pressure value | = t₂ |
| and the rate at which air is leaking into the cavity from the time the pressure in the cavity falls to the set pressure value | = L₂ litres/minute |
| then the total volume of air which would leak into the cavity during evacuating thereof from the time the cavity pressure falls to the set pressure value until the residual value of insufflating gas had been evacuated therefrom | = L₂.t₂ litres |
| thus, the corrected residual value of insufflating gas to be drawn from the cavity | = V₁ - L₁.t₁ + L₂.t₂ fitres |

Thus, the corrected residual value of insufflating gas to be drawn from the cavity from the time the cavity pressure falls to the set pressure value is stored in the memory 31 as follows: ${V}_{1} - {L}_{1} {. t}_{1} + {L}_{2} {. t}_{2}$

The reason the rate of leakage of insufflating gas from the commencement of insufflating of the cavity up to the time the cavity pressure reaches the set pressure value, is based on the rate of leakage of insufflating gas from the cavity just as the cavity has been inflated to the working pressure and is being maintained at the working pressure for a short time thereafter, and the rate of leakage of air into the cavity is based on the rate of leakage of air from the cavity while the cavity is being maintained at the working pressure for a short time just prior to termination of insufflating to the cavity, is due to the fact that in general, during insufflating of a cavity, leakage from the cavity increases from the beginning of the carrying out of the procedure to the end of the carrying out of the procedure as a result of movement of trocars and instruments into and out of the cavity during the carrying out of the procedure. Thus, it is envisaged that in general the rate of leakage of air into the cavity during evacuating of the cavity will be greater than the rate of leakage of insufflating gas from the cavity during the period from the commencement of insufflating of the cavity up to the time the cavity pressure reaches the set pressure value.

It is also envisaged that in determining the rate of leakage of insufflating gas from the cavity, if there is a significant difference between the set pressure value and the working pressure, a further correction could be made in order to determine the leakage rate of insufflating gas from the cavity at the lower set pressure value, or at an average value of the cavity pressure during the period from the commencement of insufflating of the cavity until the cavity pressure reaches the set pressure value. Similarly, the rate of leakage of air into the cavity could be corrected based on the difference between the working pressure and the set pressure value.

It is also envisaged that in some embodiments of the invention a lower pressure value below which the set pressure value should not be set may be determined. In general, it is envisaged that the lower pressure value would be determined as being the value of the transition pressure from the first pressure/volume relationship to the second pressure/volume relationship. It is also envisaged that an upper pressure value may be determined above which the set pressure value should not be set, and in general, it is envisaged that the upper pressure value would be a pressure less than the selected working pressure value. It is envisaged that at least a lower pressure value would be determined in embodiments of the invention where the set pressure value is selectable in order to assist a surgeon or clinician in determining the value at which the set pressure value should be selected. By providing an upper pressure value above which the set pressure value should not be set would also assist in manually selecting the set pressure value.

Needless to say, other suitable methods of determining the leakage of insufflating gas from the cavity during insufflating thereof, and determining the leakage of air into the cavity during evacuating of the insufflating gas from the cavity may be used.

While the set pressure value has been described as being a value equal to the transition pressure in the cavity, as the first pressure/volume relationship transitions to the second pressure/volume relationship plus a predefined pressure value, firstly, the predefined pressure value may be any suitable value, and typically, would range from a predefined pressure value of 0.1mmHg to 3mmHg, and preferably, would lie in the range from 0.5mmHg to 2mmHg. Secondly, it is also envisaged that in some embodiments of the invention the set pressure value may be determined as being the transition pressure value as the first pressure/volume relationship transitions to the second pressure/volume relationship, in other words, the point A on the graph of Fig. 2. Although, by selecting the set pressure value to be at a pressure above the transition pressure value, the value of the residual volume of insufflating gas may be determined more accurately. However, while the set pressure value may be set as being a pressure of any value along the second pressure/volume relationship, in general, it is desirable that the set pressure value be determined as a pressure closer to the transition pressure, than closer to a pressure at the point B or C on the pressure/volume relationship lines 37 and 38.

While the set pressure value has been described as being determined by the microprocessor 13, it is envisaged that in some embodiments of the invention the set pressure value may be selectable and in which case, the selected set pressure value would be entered through the touchscreen 30. However, as discussed above, the selected set pressure value should be ideally set as a pressure either equal to the transition pressure as the first pressure/volume relationship transitions to the second pressure/volume relationship, but preferably, as a pressure above the transition pressure whereby the first pressure/volume relationship transitions to the second pressure/volume relationship, and when selected above the transition pressure, the set pressure value should ideally be equal to the transition pressure value plus a predefined pressure value, typically, within the range of 0.1mmHg to 3mmHg.

While the method and insufflator have been described for insufflating a peritoneal cavity of a subject, it will be appreciated that the method and the insufflator may be used for insufflating any other cavity, lumen, vessel or organ in a human or animal subject.

While the method for determining the point of inflection, in other words, the transition pressure value, as the pressure/volume relationship transitions from the first pressure/volume relationship to the second pressure/volume relationship, has been described as comprising storing the values of the cavity pressure and the corresponding values of the cumulative volumes of insufflating gas in the cavity at the predefined time intervals of 10 milliseconds, and also has been described by computing and monitoring the increase in pressure in the cavity per unit volume of insufflating gas delivered to the cavity, it will be appreciated that any other suitable method and/or means for determining the point of inflection A, namely, the transition pressure as the first pressure/volume relationship transitions to the second pressure/volume relationship may be used. Additionally, while the values of the signals from the pressure monitoring device, the first flow rate controller and the second flow rate controller have been described as being read at predefined sampling time intervals of 10 milliseconds, the predefined sampling time intervals between reading of the values of the signals produced by the pressure monitoring device, the first flow rate sensor and the second flow rate sensor may be of any suitable or desirable time duration.

Additionally, while the pressure/volume relationship between the pressure in the cavity and the cumulative volume of insufflating gas delivered to the cavity has been described and illustrated as being represented by the first graph line 37 and by the smoothed second graph line 38, it will be appreciated by those skilled in the art that the pressure/volume relationship in other vessel, lumen, organ or cavity may vary from the graphical representations illustrated in Fig. 2. For example, in some embodiments of the invention it is envisaged that the pressure/volume relationship may be in the form of an equation, such as a quadratic equation, or other equation, which for example, may be a power law equation. In which case, the line of the equation would be determined by, for example, curve fitting, and the slope of the line of the equation would be monitored in order to determine the point of inflection of the line at which the pressure/volume relationship transitions from the first pressure/volume relationship to the second pressure/volume relationship.

While the flow sensing device has been described as comprising a first flow rate sensor for monitoring the flow rate of insufflating gas delivered to the cavity, and a second flow rate sensor for monitoring the flow rate of insufflating gas drawn from the cavity by the vacuum pump 26, it is envisaged that in some embodiments of the invention the flow sensing device may comprise a single flow sensor, through which insufflating gas being delivered to the cavity, and insufflating gas being drawn from the cavity would pass. It will also be appreciated that while the two flow sensors have been described as being flow rate sensors, in some embodiments of the invention it is envisaged that the two flow sensors may comprise flow meters, which would produce a signal indicative of the cumulative volume of insufflating gas either delivered to the cavity from the commencement of insufflating, or drawn from the cavity by the vacuum pump from a predefined time, in the case of the present invention, from the time the pressure in the cavity has fallen to the set pressure value, Similarly, in the case where a single flow sensing device is provided for monitoring the flow of insufflating gas delivered to the cavity and the flow of insufflating gas drawn from the cavity, such a single flow sensing device may also comprise a flow meter, which would produce a signal indicative of the cumulative volume of the insufflating gas either delivered to or drawn from the cavity.

While the insufflating gas has been described as comprising carbon dioxide, any other suitable insufflating gas may be used.

While the interface means has been described as comprising a touchscreen, any other suitable interface means may be provided for inputting values and data to the microprocessor, In some embodiments of the invention the insufflator may be operated remotely, for example, wirelessly from a smart mobile device, for example, a smart mobile phone, or via wireless means such as the ethernet.

It is also envisaged that in the embodiment of the invention described, since the pressure sensor is located on the trocar 6 in the cavity 3 and produces signals indicative of the pressure in the cavity 3, the pressure monitoring device may be omitted, and the signals produced by the pressure sensor would be read directly from the pressure sensor through the wire 25 by the microprocessor.

While the evacuating means for evacuating insufflating gas from the cavity has been described as comprising a vacuum pump, any other suitable evacuating means may be provided.

It will also be appreciated that in some embodiments of the invention the insufflating gas may be evacuated through a different trocar than the trocar through which the insufflating gas is delivered to the cavity, and in some embodiments of the invention, the insufflating gas may be withdrawn from the cavity though a Veress needle. In which case, it is envisaged that a separate evacuation line would be provided from the other trocar or the Veress needle to the vacuum pump through the second flow sensor.

While the microprocessor has been described as applying a smoothing algorithm to the computation of the pressure/volume relationship between the cavity pressure and the cumulative volume of insufflating gas delivered to the cavity, and to the increase in pressure in the cavity per unit volume of insufflating gas delivered to the cavity, in some embodiments of the invention it is envisaged that a smoothing algorithm may not be applied to the computations of the pressure/volume relationship and the increase in pressure in the cavity per unit volume of insufflating gas delivered to the cavity. Additionally, it is envisaged that in cases where a smoothing algorithm is applied to the computations, any other suitable smoothing algorithm may be applied to the computations besides a moving average algorithm.

While the pressure regulator has been described as stepping down the pressure of the insufflating gas to 40mmHg, it will be appreciated by those skilled in the art that the pressure regulator may step the pressure of the insufflating gas down to any suitable pressure, and in some embodiments of the invention the pressure to which the insufflating gas is stepped down by the pressure regulator may be less than or greater than 40mmHg. Indeed, in some embodiments of the invention the pressure regulator may be configured to step down the pressure of the insufflating gas to a pressure significantly higher than the pressure of 40mmHg, and in some cases, may only reduce the pressure of the insufflating gas to a pressure in the order of 3.5bar. In which case, the flow controller would be configured to reduce the pressure from such a pressure to a suitable pressure, such that the insufflating gas would be supplied to the cavity at the appropriate pressure, such that during the procedure, the pressure in the cavity would be maintained at the selected working pressure, or at any other pressure chosen or selected by the surgeon or clinician. During initial insufflating of the cavity in order to determine the set pressure value, the flow controller would be operated to supply the insufflating gas to the cavity at the appropriate constant rate.

## Claims

1. An insufflator for insufflating a cavity in the body of a human or animal subject, the insufflator comprising:
a delivery means (16) for delivering insufflating gas to the cavity for insufflating the cavity at a working pressure value,
a pressure sensor (23, 24) for monitoring pressure in the cavity and for producing a signal indicative of the pressure in the cavity,
a flow sensing device (21) for monitoring flow of insufflating gas delivered to the cavity and for producing a signal indicative of the cumulative volume of insufflating gas delivered to the cavity from the commencement of delivery of the insufflating gas thereto or for producing a signal from which the cumulative volume of insufflating gas delivered to the cavity from the commencement of delivery of the insufflating gas thereto may be determined, and
a signal processor (13) programmed:
to operate the delivery means (16) to deliver insufflating gas to the cavity for insufflating thereof, and
to read signals from the pressure sensor (23, 24) and from the flow sensing device (21) during insufflating of the cavity,
**characterised in that** the signal processor (13) is programmed:
to read signals from the pressure sensor (23, 24) and from the flow sensing device (21) from the commencement of insufflating of the cavity until the cavity pressure reaches a set pressure value, the set pressure value being less than the working pressure value,
to determine from the signals read from the pressure sensor (23, 24) and the flow sensing device (21) from the commencement of insufflating of the cavity a value of an initial volume of insufflating gas delivered to the cavity, the initial volume of insufflating gas delivered to the cavity being the cumulative volume of insufflating gas delivered to the cavity from the commencement of delivery of the insufflating gas thereto until the cavity pressure reaches the set pressure value, and
to determine a value of a residual volume of insufflating gas to be drawn from the cavity during evacuating of insufflating gas from the cavity during termination of insufflating thereof, the value of the residual volume being determined as a function of the determined value of the initial volume of insufflating gas delivered to the cavity, so that during evacuating of insufflating gas from the cavity during termination of insufflating thereof, on the cavity pressure falling to the set pressure value, by evacuating a further volume of insufflating gas from the cavity substantially equal to the value of the residual volume thereof, substantially no insufflating gas remains in the cavity on completion of evacuating thereof.

2. An insufflator as claimed in Claim 1 **characterised in that** the signal processor (13) is programmed to control the delivery means (16) to deliver the insufflating gas to the cavity at a substantially constant rate until the value of the initial volume of the insufflating gas has been determined.

3. An insufflator as claimed in Claim 1 or 2 **characterised in that** the signal processor (13) is programmed, on the value of the initial volume of the insufflating gas being determined, to control the delivery means (16) to deliver the insufflating gas to the cavity at a flow rate to insufflate the cavity to the working pressure value and to maintain the pressure in the cavity at the working pressure value.

4. An insufflator as claimed in any preceding claim **characterised in that** the insufflator further comprises an evacuating means (26) for evacuating insufflating gas from the cavity, the signal processor (13) being programmed to control the operation of the evacuating means (26) for evacuating insufflating gas from the cavity.

5. An insufflator as claimed in Claim 4 **characterised in that** the signal processor (13) is programmed
to determine when the pressure in the cavity falls to the set pressure value from the signal read from the pressure sensor (23, 24) during evacuating of insufflating gas from the cavity,
to determine the cumulative volume of insufflating gas evacuated from the cavity from the time the pressure in the cavity falls to the set pressure value from the signal read from the flow sensing device (22), and
to terminate operation of the evacuating means (26) on the determined cumulative volume of insufflating gas evacuated from the cavity from the time the pressure falls to the set pressure value being equal to the determined value of the residual volume of the insufflating gas.

6. An insufflator as claimed in any preceding claim **characterised in that** the signal processor (13) is programmed to determine the value of the residual volume of the insufflating gas as being a function of the value of the determined initial volume of insufflating gas delivered to the cavity from the commencement of insufflating thereof until the pressure in the cavity reaches the set pressure value and as a function of one or both of a compensating factor or a compensating constant.

7. An insufflator as claimed in Claim 6 **characterised in that** the one or both of the compensating factor and the compensating constant are selected to compensate for one or both of any leakage of insufflating gas from the cavity during the insufflating of the cavity until the pressure in the cavity reaches the set pressure value and/or any leakage of air being drawn into the cavity during evacuating thereof after the pressure in the cavity has fallen to the set pressure value.

8. An insufflator as claimed in any preceding claim **characterised in that** the signal processor (13) is programmed to determine a transition pressure value (45) at which a first pressure/volume relationship (40, 43) transitions to a subsequent second pressure/volume relationship (41, 44) of a pressure/volume relationship (37, 38) between the pressure in the cavity and the cumulative volume of insufflating gas delivered to the cavity from the commencement of delivery thereof, the second pressure/volume relationship (41, 44) being different to the first pressure/volume relationship (40, 43).

9. An insufflator as claimed in Claim 8 **characterised in that** the signal processor (13) is programmed to determine the first pressure/volume relationship (40, 43) as a part of the pressure/volume relationship (37, 38) during which the pressure in the cavity remains substantially constant as insufflating gas is being delivered to the cavity, and to determine the second pressure/volume relationship (41, 44) as a subsequent part of the pressure/volume relationship (37, 38) during which the pressure in the cavity increases linearly with respect to the delivery of insufflating gas to the cavity.

10. An insufflator as claimed in Claim 8 or 9 **characterised in that** the signal processor (13) is programmed to determine the transition pressure value (45) as the value of the pressure in the cavity at the end of the first pressure/volume relationship (40, 43), as the value of the pressure in the cavity at the commencement of the second pressure/volume relationship (41,44), or as a pressure value between the value of the pressure in the cavity at the end of the first pressure/volume relationship (40, 43) and the pressure in the cavity at the commencement of the second pressure/volume relationship (41, 44).

11. An insufflator as claimed in any of Claims 8 to 10 **characterised in that** the signal processor (13) is programmed to determine a lower pressure value below which the set pressure value should not be set as being a pressure value not less than or equal to the pressure in the cavity at the end of the first pressure/volume relationship (40, 43).

12. An insufflator as claimed in any of Claims 8 to 11 **characterised in that** the signal processor (13) is programmed to determine the set pressure value.

13. An insufflator as claimed in Claim 12 **characterised in that** the signal processor (13) is programmed to determine the set pressure value at a pressure value equal to the determined transition pressure value or a value equal to the sum of the determined transition pressure value and a predefined pressure value.

14. An insufflator as claimed in Claim 13 **characterised in that** the predefined pressure value lies in the range of 0.1mmHg to 3mmHg.

15. An insufflator as claimed in any of Claims 1 to 11 **characterised in that** the set pressure value is selectable.

## Patentansprüche

1. Insufflator zum Insufflieren einer Kavität im Körper eines menschlichen oder tierischen Subjekts, wobei der Insufflator umfasst:
ein Zuführmittel (16) zum Zuführen von Insufflationsgas zu der Kavität zum Insufflieren der Kavität auf einen Arbeitsdruckwert,
einen Drucksensor (23, 24) zum Überwachen des Drucks in der Kavität und zum Erzeugen eines Signals, das den Druck in der Kavität angibt,
eine Durchflusserfassungseinrichtung (21) zum Überwachen des Durchflusses des zu der Kavität zugeführten Insufflationsgases und zum Erzeugen eines Signals, das das kumulative Volumen des der Kavität ab Beginn der Zuführung des Insufflationsgases zu derselben zugeführten Insufflationsgases angibt, oder zum Erzeugen eines Signals, aus dem das kumulative Volumen des der Kavität ab Beginn der Zuführung des Insufflationsgases zu derselben zugeführten Insufflationsgases bestimmt werden kann, und
einen Signalprozessor (13), der programmiert ist zum:
Betreiben des Zuführmittels (16), um der Kavität Insufflationsgas zum Insufflieren derselben zuzuführen, und
Lesen von Signalen von dem Drucksensor (23, 24) und von der Durchflusserfassungseinrichtung (21) während des Insufflierens der Kavität, **dadurch gekennzeichnet, dass** der Signalprozessor (13) programmiert ist zum:
Lesen von Signalen von dem Drucksensor (23, 24) und von der Durchflusserfassungseinrichtung (21) ab Beginn des Insufflierens der Kavität, bis der Druck in der Kavität einen festgelegten Druckwert erreicht, wobei der festgelegte Druckwert kleiner als der Arbeitsdruckwert ist,
Bestimmen eines Werts eines der Kavität zugeführten Anfangsvolumens an Insufflationsgas aus den von dem Drucksensor (23, 24) und der Durchflusserfassungseinrichtung (21) ab Beginn des Insufflierens der Kavität gelesenen Signalen, wobei das der Kavität zugeführte Anfangsvolumen an Insufflationsgas das kumulative Volumen des Insufflationsgases ist, das der Kavität ab Beginn der Zuführung des Insufflationsgases bis zum Erreichen des festgelegten Druckwerts in der Kavität zugeführt wird, und
Bestimmen eines Werts eines Restvolumens an Insufflationsgas, das während des Evakuierens von Insufflationsgas aus der Kavität im Zuge der Beendigung des Insufflierens derselben aus der Kavität abzusaugen ist, wobei der Wert des Restvolumens als Funktion des bestimmten Werts des der Kavität zugeführten Anfangsvolumens an Insufflationsgas bestimmt wird, sodass während des Evakuierens von Insufflationsgas aus der Kavität im Zuge der Beendigung des Insufflierens derselben, wenn der Druck in der Kavität auf den festgelegten Druckwert fällt, durch Evakuieren eines weiteren Volumens an Insufflationsgas aus der Kavität, das im Wesentlichen gleich dem Wert des Restvolumens desselben ist, nach Abschluss des Evakuierens derselben im Wesentlichen kein Insufflationsgas in der Kavität verbleibt.

2. Insufflator nach Anspruch 1, **dadurch gekennzeichnet, dass** der Signalprozessor (13) dazu programmiert ist, das Zuführmittel (16) derart zu steuern, dass es der Kavität das Insufflationsgas mit einer im Wesentlichen konstanten Rate zuführt, bis der Wert des Anfangsvolumens des Insufflationsgases bestimmt worden ist.

3. Insufflator nach Anspruch 1 oder 2, **dadurch gekennzeichnet, dass** der Signalprozessor (13) dazu programmiert ist, wenn der Wert des Anfangsvolumens des Insufflationsgases bestimmt worden ist, das Zuführmittel (16) derart zu steuern, dass es der Kavität das Insufflationsgas mit einer Durchflussrate zuführt, um die Kavität auf den Arbeitsdruckwert zu insufflieren und den Druck in der Kavität auf dem Arbeitsdruckwert zu halten.

4. Insufflator nach einem der vorhergehenden Ansprüche, **dadurch gekennzeichnet, dass** der Insufflator ferner ein Evakuierungsmittel (26) zum Evakuieren von Insufflationsgas aus der Kavität umfasst, wobei der Signalprozessor (13) dazu programmiert ist, den Betrieb des Evakuierungsmittels (26) zum Evakuieren von Insufflationsgas aus der Kavität zu steuern.

5. Insufflator nach Anspruch 4, **dadurch gekennzeichnet, dass** der Signalprozessor (13) programmiert ist zum:
Bestimmen, wann der Druck in der Kavität auf den festgelegten Druckwert fällt, anhand des während des Evakuierens von Insufflationsgas aus der Kavität von dem Drucksensor (23, 24) gelesenen Signals,
Bestimmen des kumulativen Volumens des aus der Kavität evakuierten Insufflationsgases ab dem Zeitpunkt, zu dem der Druck in der Kavität auf den festgelegten Druckwert fällt, anhand des von der Durchflusserfassungseinrichtung (22) gelesenen Signals, und
Beenden des Betriebs des Evakuierungsmittels (26), wenn das bestimmte kumulative Volumen des Insufflationsgases, das ab dem Zeitpunkt, zu dem der Druck auf den festgelegten Druckwert fällt, aus der Kavität evakuiert wird, gleich dem bestimmten Wert des Restvolumens des Insufflationsgases ist.

6. Insufflator nach einem der vorhergehenden Ansprüche, **dadurch gekennzeichnet, dass** der Signalprozessor (13) dazu programmiert ist, den Wert des Restvolumens des Insufflationsgases als Funktion des Werts des bestimmten Anfangsvolumens an Insufflationsgas, das der Kavität ab Beginn des Insufflierens derselben bis zum Erreichen des festgelegten Druckwerts zugeführt wird, und als Funktion eines Kompensationsfaktors und/oder einer Kompensationskonstante zu bestimmen.

7. Insufflator nach Anspruch 6, **dadurch gekennzeichnet, dass** der Kompensationsfaktor und/oder die Kompensationskonstante ausgewählt sind, um eine etwaige Leckage von Insufflationsgas aus der Kavität während des Insufflierens der Kavität, bis der Druck in der Kavität den festgelegten Druckwert erreicht, und/oder eine etwaige Leckage von Luft, die während des Evakuierens derselben in die Kavität eingesaugt wird, nachdem der Druck in der Kavität auf den festgelegten Druckwert gefallen ist, zu kompensieren.

8. Insufflator nach einem der vorhergehenden Ansprüche, **dadurch gekennzeichnet, dass** der Signalprozessor (13) dazu programmiert ist, einen Übergangsdruckwert (45) zu bestimmen, bei dem eine erste Druck/Volumen-Beziehung (40, 43) in eine nachfolgende zweite Druck/Volumen-Beziehung (41, 44) einer Druck/Volumen-Beziehung (37, 38) zwischen dem Druck in der Kavität und dem kumulativen Volumen des der Kavität ab Beginn seiner Zuführung zugeführten Insufflationsgases übergeht, wobei sich die zweite Druck/Volumen-Beziehung (41, 44) von der ersten Druck/Volumen-Beziehung (40, 43) unterscheidet.

9. Insufflator nach Anspruch 8, **dadurch gekennzeichnet, dass** der Signalprozessor (13) dazu programmiert ist, die erste Druck/Volumen-Beziehung (40, 43) als einen Teil der Druck/Volumen-Beziehung (37, 38) zu bestimmen, während dessen der Druck in der Kavität im Wesentlichen konstant bleibt, während der Kavität Insufflationsgas zugeführt wird, und die zweite Druck/Volumen-Beziehung (41, 44) als einen nachfolgenden Teil der Druck/Volumen-Beziehung (37, 38) zu bestimmen, während dessen der Druck in der Kavität in Bezug auf die Zuführung von Insufflationsgas zu der Kavität linear ansteigt.

10. Insufflator nach Anspruch 8 oder 9, **dadurch gekennzeichnet, dass** der Signalprozessor (13) dazu programmiert ist, den Übergangsdruckwert (45) als den Wert des Drucks in der Kavität am Ende der ersten Druck/Volumen-Beziehung (40, 43), als den Wert des Drucks in der Kavität zu Beginn der zweiten Druck/Volumen-Beziehung (41, 44) oder als einen Druckwert zwischen dem Wert des Drucks in der Kavität am Ende der ersten Druck/Volumen-Beziehung (40, 43) und dem Druck in der Kavität zu Beginn der zweiten Druck/Volumen-Beziehung (41, 44) zu bestimmen.

11. Insufflator nach einem der Ansprüche 8 bis 10, **dadurch gekennzeichnet, dass** der Signalprozessor (13) dazu programmiert ist, einen unteren Druckwert, unterhalb dessen der festgelegte Druckwert nicht eingestellt werden sollte, als einen Druckwert zu bestimmen, der nicht kleiner als oder gleich dem Druck in der Kavität am Ende der ersten Druck/Volumen-Beziehung (40, 43) ist.

12. Insufflator nach einem der Ansprüche 8 bis 11, **dadurch gekennzeichnet, dass** der Signalprozessor (13) dazu programmiert ist, den festgelegten Druckwert zu bestimmen.

13. Insufflator nach Anspruch 12, **dadurch gekennzeichnet, dass** der Signalprozessor (13) dazu programmiert ist, den festgelegten Druckwert als einen Druckwert, der gleich dem bestimmten Übergangsdruckwert ist, oder als einen Wert zu bestimmen, der gleich der Summe aus dem bestimmten Übergangsdruckwert und einem vordefinierten Druckwert ist.

14. Insufflator nach Anspruch 13, **dadurch gekennzeichnet, dass** der vordefinierte Druckwert im Bereich von 0,1 mmHg bis 3 mmHg liegt.

15. Insufflator nach einem der Ansprüche 1 bis 11, **dadurch gekennzeichnet, dass** der festgelegte Druckwert auswählbar ist.

## Revendications

1. Insufflateur pour insuffler une cavité dans le corps d'un sujet humain ou animal, l'insufflateur comprenant :
un moyen d'alimentation (16) pour délivrer du gaz d'insufflation à la cavité pour insuffler la cavité à une valeur de pression de service,
un capteur de pression (23, 24) pour surveiller la pression dans la cavité et pour produire un signal indicatif de la pression dans la cavité,
un dispositif de détection de débit (21) pour surveiller le débit de gaz d'insufflation délivré à la cavité et pour produire un signal indicatif du volume cumulé de gaz d'insufflation délivré à la cavité depuis le début de l'alimentation du gaz d'insufflation à celle-ci, ou pour produire un signal à partir duquel le volume cumulé de gaz d'insufflation délivré à la cavité depuis le début de l'alimentation du gaz d'insufflation à celle-ci peut être déterminé, et
un processeur de signaux (13) programmé :
pour actionner le moyen d'alimentation (16) pour délivrer du gaz d'insufflation à la cavité pour l'insuffler, et
pour lire des signaux provenant du capteur de pression (23, 24) et du dispositif de détection de débit (21) pendant l'insufflation de la cavité, **caractérisé en ce que** le processeur de signaux (13) est programmé :
pour lire des signaux provenant du capteur de pression (23, 24) et du dispositif de détection de débit (21) depuis le début de l'insufflation de la cavité jusqu'à ce que la pression dans la cavité atteigne une valeur de pression de consigne, la valeur de pression de consigne étant inférieure à la valeur de pression de service,
pour déterminer, à partir des signaux lus à partir du capteur de pression (23, 24) et du dispositif de détection de débit (21) dès le début de l'insufflation de la cavité, une valeur d'un volume initial de gaz d'insufflation délivré à la cavité, le volume initial de gaz d'insufflation délivré à la cavité étant le volume cumulé de gaz d'insufflation délivré à la cavité depuis le début de l'alimentation du gaz d'insufflation à celle-ci jusqu'à ce que la pression dans la cavité atteigne la valeur de pression de consigne, et
pour déterminer une valeur du volume résiduel de gaz d'insufflation à extraire de la cavité lors de l'évacuation du gaz d'insufflation de la cavité lors de l'arrêt de l'insufflation de celle-ci, la valeur du volume résiduel étant déterminée en fonction de la valeur déterminée du volume initial de gaz d'insufflation délivré à la cavité, de sorte que, lors de l'évacuation du gaz d'insufflation de la cavité lors de l'arrêt de l'insufflation de celle-ci, lorsque la pression dans la cavité chute jusqu'à la valeur de pression de consigne, en évacuant de la cavité un volume supplémentaire de gaz d'insufflation sensiblement égal à la valeur du volume résiduel de celle-ci, il ne reste pratiquement plus de gaz d'insufflation dans la cavité à l'issue de l'évacuation de celle-ci.

2. Insufflateur selon la revendication 1, **caractérisé en ce que** le processeur de signaux (13) est programmé pour contrôler le moyen d'alimentation (16) pour délivrer le gaz d'insufflation dans la cavité à un débit sensiblement constant jusqu'à ce que la valeur du volume initial du gaz d'insufflation ait été déterminée.

3. Insufflateur selon la revendication 1 ou 2, **caractérisé en ce que** le processeur de signaux (13) est programmé, une fois la valeur du volume initial du gaz d'insufflation déterminée, pour contrôler le moyen d'alimentation (16) pour délivrer le gaz d'insufflation dans la cavité à un débit permettant d'insuffler la cavité jusqu'à la valeur de pression de service et pour maintenir la pression dans la cavité à la valeur de pression de service.

4. Insufflateur selon l'une quelconque des revendications précédentes, **caractérisé en ce que** l'insufflateur comprend en outre un moyen d'évacuation (26) pour évacuer le gaz d'insufflation de la cavité, le processeur de signaux (13) étant programmé pour contrôler le fonctionnement du moyen d'évacuation (26) pour évacuer le gaz d'insufflation de la cavité.

5. Insufflateur selon la revendication 4, **caractérisé en ce que** le processeur de signaux (13) est programmé
pour déterminer, à partir du signal lu à partir du capteur de pression (23, 24) pendant l'évacuation du gaz d'insufflation de la cavité, le moment où la pression dans la cavité chute à la valeur de pression de consigne,
pour déterminer le volume cumulé de gaz d'insufflation évacué de la cavité à partir du moment où la pression dans la cavité atteint la valeur de pression de consigne, à partir du signal lu à partir du dispositif de détection de débit (22), et
pour interrompre le fonctionnement du moyen d'évacuation (26) lorsque le volume cumulé déterminé de gaz d'insufflation évacué de la cavité depuis le moment où la pression est tombée à la valeur de pression de consigne est égal à la valeur déterminée du volume résiduel du gaz d'insufflation.

6. Insufflateur selon l'une quelconque des revendications précédentes, **caractérisé en ce que** le processeur de signaux (13) est programmé pour déterminer la valeur du volume résiduel du gaz d'insufflation en fonction de la valeur du volume initial déterminé de gaz d'insufflation délivré dans la cavité depuis le début de l'insufflation de celle-ci jusqu'à ce que la pression dans la cavité atteigne la valeur de pression de consigne, et en fonction d'un facteur de compensation ou d'une constante de compensation, ou des deux.

7. Insufflateur selon la revendication 6, **caractérisé en ce que** le facteur de compensation ou la constante de compensation, ou les deux, sont choisis pour compenser l'une ou l'autre, ou les deux, des fuites de gaz d'insufflation hors de la cavité pendant l'insufflation de la cavité jusqu'à ce que la pression dans la cavité atteigne la valeur de pression de consigne, et/ou des fuites d'air aspiré dans la cavité pendant l'évacuation de celle-ci une fois que la pression dans la cavité est tombée à la valeur de pression de consigne.

8. Insufflateur selon l'une quelconque des revendications précédentes, **caractérisé en ce que** le processeur de signaux (13) est programmé pour déterminer une valeur de pression de transition (45) à laquelle une première relation pression/volume (40, 43) passe à une deuxième relation pression/volume (41, 44) d'une relation pression/volume (37, 38) entre la pression dans la cavité et le volume cumulé de gaz d'insufflation délivré à la cavité depuis le début de l'alimentation à celle-ci, la deuxième relation pression/volume (41, 44) étant différente de la première relation pression/volume (40, 43).

9. Insufflateur selon la revendication 8, **caractérisé en ce que** le processeur de signaux (13) est programmé pour déterminer la première relation pression/volume (40, 43) en tant que partie de la relation pression/volume (37, 38) au cours de laquelle la pression dans la cavité reste sensiblement constante pendant que le gaz d'insufflation est délivré à la cavité, et pour déterminer la deuxième relation pression/volume (41, 44) comme une partie subséquente de la relation pression/volume (37, 38) au cours de laquelle la pression dans la cavité augmente linéairement en fonction de l'alimentation de gaz d'insufflation à la cavité.

10. Insufflateur selon la revendication 8 ou 9, **caractérisé en ce que** le processeur de signaux (13) est programmé pour déterminer la valeur de pression de transition (45) comme étant la valeur de la pression dans la cavité à la fin de la première relation pression/volume (40, 43), comme étant la valeur de la pression dans la cavité au début de la deuxième relation pression/volume (41, 44), ou comme étant une valeur de pression comprise entre la valeur de la pression dans la cavité à la fin de la première relation pression/volume (40, 43) et la pression dans la cavité au début de la deuxième relation pression/volume (41, 44).

11. Insufflateur selon l'une quelconque des revendications 8 à 10, **caractérisé en ce que** le processeur de signaux (13) est programmé pour déterminer une valeur de pression inférieure en dessous de laquelle la valeur de pression de consigne ne doit pas être réglée, cette valeur étant une valeur de pression non inférieure ou égale à la pression dans la cavité à la fin de la première relation pression/volume (40, 43).

12. Insufflateur selon l'une quelconque des revendications 8 à 11, **caractérisé en ce que** le processeur de signaux (13) est programmé pour déterminer la valeur de pression de consigne.

13. Insufflateur selon la revendication 12, **caractérisé en ce que** le processeur de signaux (13) est programmé pour déterminer la valeur de pression de consigne à une valeur de pression égale à la valeur de pression de transition déterminée ou à une valeur égale à la somme de la valeur de pression de transition déterminée et d'une valeur de pression prédéfinie.

14. Insufflateur selon la revendication 13, **caractérisé en ce que** la valeur de pression prédéfinie se situe dans la plage de 0,1 mmHg à 3 mmHg.

15. Insufflateur selon l'une quelconque des revendications 1 à 11, **caractérisé en ce que** la valeur de pression de consigne est sélectionnable.
